Europäisches Patentamt

European Patent Office

Office européen des brevets

(11) Veröffentlichungsnummer: **0 256 321**

**A1**

(12) **EUROPÄISCHE PATENTANMELDUNG**

(21) Anmeldenummer: **87110347.9**

(22) Anmeldetag: **17.07.87**

(51) Int. Cl.⁴ **C12N 15/00** , C12Q 1/70 ,
C12P 21/02 , C12P 21/00 ,
G01N 33/569 , G01N 33/571 ,
A61K 39/12

(30) Priorität: **23.07.86 DE 3624786**
**25.07.86 DE 3625257**

(43) Veröffentlichungstag der Anmeldung:
**24.02.88 Patentblatt 88/08**

(84) Benannte Vertragsstaaten:
**AT BE CH DE FR GB IT LI LU NL SE**

(71) Anmelder: **BEHRINGWERKE**
**Aktiengesellschaft**
**Postfach 1140**
**D-3550 Marburg 1(DE)**

(72) Erfinder: **Oltersdorf, Tilmann, Dr.**
**St.-Anna-Gasse 13**
**D-6900 Heidelberg(DE)**
Erfinder: **Röwekamp, Walter, Dr.**
**Emmertsgrundpassage 11**
**D-6900 Heidelberg(DE)**
Erfinder: **Schneider-Gädicke, Ansbert, Dr.**
**Handschuhsheimer Landstrasse 64**
**D-6900 Heidelberg(DE)**
Erfinder: **Seedorf, Klaus**
**Albert-Schweitzer-Strasse 41**
**D-6803 Edingen(DE)**
Erfinder: **Dürst, Matthias, Dr.**
**Neuenheimer Landstrasse 72**
**D-6900 Heidelberg(DE)**
Erfinder: **Schwarz, Elisabeth, Dr.**
**Schröderstrasse 37**
**D-6900 Heidelberg(DE)**

(74) Vertreter: **Meyer-Dulheuer, Karl-Hermann, Dr.**
**et al**
**HOECHST Aktiengesellschaft Zentrale**
**Patentabteilung Postfach 80 03 20**
**D-6230 Frankfurt/Main 80(DE)**

(54) Expressionsprodukte des menschilchen Papillomvirus Typ 18, für diese Proteine spezifische Antikörper und diese Antikörper bzw. entsprechende DNA enthaltende Diagnostika.

(57) Durch die teilweise Charakterisierung des Genoms und der cDNA von HPV18 können mit gentechnologischer Expression der offenen Leseraster die HPV18 Proteine erhalten werden. Die besonders geeigneten Expressionsplasmide pEX8-mer, pEX10-mer und pEX-12-mer erlauben die Isolierung dieser HPV18-Proteine in Mengen, die die Gewinnung von poly-oder monoklonalen Antikörpern ermöglicht.

**Expressionsprodukte des menschlichen Papillomvirus Typ 18, für diese Proteine spezifische Antikörper und diese Antikörper bzw. entsprechende DNA enthaltende Diagnostika**

Dürst et al., Proc. Natl. Acad. Sci. USA 80 (1983) 3813 -3815 beschreiben einen neuen Typ von menschlichen Papillomviren (HPV), den sie als HPV16 bezeichnen. Die DNA-Sequenz und die Genom-Organisation dieses Virus sind in Seedorf et al., Virology 145 (1985) 181 - 185 wiedergegeben. In Boshart et al., EMBO J. 3 (1984) 1151 -1157 ist ein weiterer neuer humaner Papillomvirus-Typ beschrieben, der als HPV18 definiert wird. In dieser Literaturstelle wird auch auf die Ähnlichkeit von HPV16 und HPV18 verwiesen, insbesondere auf die Tatsache, daß beide mit malignen genitalen Tumoren verbunden sind. Die Genomgröße ist mit etwa 7,9 kb in der gleichen Größenordnung und die Genom-Organisation ist gleich.

Papillomviren lassen sich nicht in Kultur vermehren. Die Verwendung der HPV18-DNA als Diagnostikum sowie die Gewinnung der Expressionsprodukte, deren Verwendung als Antigene, die Isolierung von Antikörpern und die Herstellung entsprechender Diagnostika setzt somit gentechnische Verfahren voraus.

Der Erfindung liegt die teilweise Charakterisierung des Genoms und der cDNA von HPV18 zugrunde. Hierdurch wird ein Zugang zur Frühdiagnose von Genitaltumoren eröffnet, die mit HPV18 assoziiert sind.

Die Erfindung ist in den Patentansprüchen definiert. Weitere Ausgestaltungen der Erfindung sind im folgenden näher beschrieben.

Die DNA-Sequenz I zeigt die ersten 1720 bp von HPV18. Die Expressionsprodukte E6, E7 und E1 sind entsprechend markiert. Die Leseraster sind jeweils am rechten Rand angegeben. Eckige Klammern geben die Position von "splice donor" und "splice acceptor sites" an.

Durch teilweises Sequenzieren eines 2,7 kb Xba I-Fragmentes und Homologievergleich zu HPV16 konnte die in der folgenden Tabelle wiedergegebene Nuklein-und Aminosäuresequenz vom offenen Leseraster L1 des HPV18-Genoms ermittelt werden. Die Homologien zu HPV16 liegen zwischen den Positionen 5754 und 5871 der DNA-Sequenz von HPV16.

Tabelle 1

|  | Ser | Arg | Leu | Leu | Thr | Val | Gly | Asn | Pro | Tyr | Phe | Arg | Val |
|---|---|---|---|---|---|---|---|---|---|---|---|---|---|
| HPV18 | TCT | AGA | TTA | TTA | ACT | GTT | GGT | AAT | CCA | TAT | TTT | AGG | GTT |
| HPV16 | TCC | AGA | CTA | CTT | GCA | GTT | GGA | CAT | CCC | TAT | TTT | CCT | ATT |
|  | Ser | Arg | Leu | Leu | Ala | Val | Gly | His | Pro | Tyr | Phe | Pro | Ile |

| Pro | Ala | Gly | Gly | Gly | Asn | Lys | Gln | Asp | Ile | Pro | Lys | Val | Ser | Ala |
|---|---|---|---|---|---|---|---|---|---|---|---|---|---|---|
| CCT | GCA | GGT | GGT | GGC | AAT | AAG | CAG | GAT | ATT | CCT | AAG | GTT | TCT | GCA |
| AAA | AAA | CCT | AAC | AAT | AAC | AAA | ATA | TTA | GTT | CCT | AAA | GTA | TCA | GGA |
| Lys | Lys | Pro | Asn | Asn | Asn | Lys | Ile | Leu | Val | Pro | Lys | Val | Ser | Gly |

| Tyr | Gln | Tyr | Arg | Val | Phe | Arg | Val | Gln | Leu | Pro |
|---|---|---|---|---|---|---|---|---|---|---|
| TAC | CAA | TAT | AGA | GTA | TTT | AGG | GTG | CAG | TTA | CCT |
| TTA | CAA | TAC | AGG | GTA | TTT | AGA | ATA | CAT | TTA | CCT |
| Leu | Gln | Tyr | Arg | Val | Phe | Arg | Ile | His | Leu | Pro |

In Kenntnis dieser DNA-Sequenz kann ein Durchschnittsfachmann ohne weiteres die gesamte DNA-Sequenz ermitteln, die für das Protein L1 von HPV18 codiert.

Weiterhin ist es in Kenntnis der hier offenbarten DNA-Teilsequenzen von HPV18 sowie aufgrund der Tatsache, daß die Genom-Organisation mit HPV16 übereinstimmt, gelungen, die gesamte DNA von HPV18 aus einer geeigneten Genbank, wie sie beispielsweise von Dürst et al. und Boshart et al. beschrieben sind, zu isolieren und zu charakterisieren. Außer den bei Boshart et al. genannten Zellinien (HeLa, KB, C4-1) kommt auch SW 756 als Basis für eine genomische oder cDNA-Genbank in Betracht. Insbesondere in der HeLa-Zellinie ist das HPV18-Genom in hoher Kopienzahl integriert.

Die DNA-Sequenz II zeigt die cDNA-Sequenz, die aus mRNA von HeLa-Zellen erhalten wurde, im Bereich der offenen Leseraster E6 bzw. E6ˉ, E7 und E1 (Anfang). Pfeile zeigen die "splice sites" an. Das ausgeschnittene E6-Intron ist durch eine dünne Linie markiert. Cystein-Reste sind durch Umrahmung hervorgehoben.

Durch die Kenntnis der Leserastergrenzen von HPV16 und 18 können in an sich bekannter Weise die entsprechenden DNA-Sequenzen in üblichen Expressionsystemen exprimiert und die viralen Proteine gewonnen werden. Besonders bewährt haben sich Expressionsvektoren vom Typ pPLc24 (Remaut et al., Gene 15 (1981) 81 - 93, 22 (1983) 103 - 113, Nucl. Acids Res. 11 (1983) 4677 - 4688). Durch Einfügen eines Polylinkers hinter dem MS-2-Polymeraseanteil sowie eines 8-, 10-bzw. 12-mer-EcoRI-Linkers (in die Smal-Stelle dieses Polylinkers) entstehen aus pPLc24 die Expressionsvektoren pEX 8-mer, pEX 10-mer und pEX 12-mer, die problemlos die Klonierung von Fragmenten im richtigen Leseraster erlauben. Man erhält damit Fusionsproteine aus einem MS-2-Polymeraseanteil und dem codierten viralen Protein bzw. Proteinteil. In E. coli finden sich diese als unlösliche Fusionsproteine nach Zellaufschluß in der Membranfraktion, was die Aufarbeitung und Reinigung sehr erleichtert. Die so erhaltenen Proteine können unmittelbar als Antigene zur Immunisierung von Versuchstieren eingesetzt werden.

Beispiel 1: Vektorkonstruktion

Der Expressionsvektor pPLc24 (Remaut et al., 1981, a.a.O.) wird zunächst mit EcoRI geöffnet, die überstehenden Enden mit Hilfe von Klenow-Enzym aufgefüllt und die stumpfen Enden religiert. Hierdurch wird die EcoRI-Schnittstelle eliminiert. Der so modifizierte Vektor wird dann mit BamHI und HindIII geöffnet und in diese Stelle eine Polylinkersequenz kloniert.

Hierzu wird das Plasmid pUC8 mit EcoRI geöffnet, die Enden aufgefüllt und ein BglII-Linker eingesetzt. Nun wird das BglII-HindIII-Polylinker-Fragment herausgeschnitten, das in das modifizierte pPLc24 eingesetzt wird.

Der resultierende Vektor wird nun mit Smal geöffnet und in die Schnittstelle einer der drei in der folgenden Tabelle aufgeführten (stumpfendigen) Polylinker eingefügt. Man erhält so die Expressionsvektoren pEX 8-mer, pEX 10-mer und pEX 12-mer. Diese Vektoren unterscheiden sich nur dadurch, daß die Polylinkerregion BamHI-SalI-PstI-HindIII um jeweils eine Position im Leseraster verschoben ist.

| pEX8-mer | Leu | Asp | Ser | Gly | Asp | Pro | Ser | Ser | Cys | Ser | Glu | Ala | Trp |
|---|---|---|---|---|---|---|---|---|---|---|---|---|---|
| | CTG | AAT | TCC | GGG | GAT | CCG | TCG | ACC | TGC | AGC | CAA | GCT | TGG |

| pEX10-mer | Leu | Asp | Ser | Gly | Gly | Ser | Val | Asp | Leu | Glu | Thr | Ser | Leu |
|---|---|---|---|---|---|---|---|---|---|---|---|---|---|
| | CTG | AAT | TCC | GGG | GGA | TCC | GTC | GAC | CTG | CAG | CCA | AGC | TTG |

| pEX12-mer | Leu | Asp | Ser | Gly | Gly | Ile | Arg | Arg | Pro | Ala | Ala | Lys | Leu |
|---|---|---|---|---|---|---|---|---|---|---|---|---|---|
| | CTG | AAT | TCC | GGG | GGA | ATC | CGT | CGA | CCT | GCA | GCC | AAG | CTT |

EcoRI      NciI      BamHI      SalI      PstI      HindIII

Diese Vektoren codieren somit für Fusionsproteine, bei denen zunächst die ersten 100 Aminosäuren der MS-2-Polymerase vorliegen, worauf das Brückenglied entsprechend der Polylinkersequenz folgt, woran sich das gewünschte Protein - im vorliegenden Falle also ein virales Protein - anschließt.

Im folgenden wird beispielhaft ein Expressionsvektor beschrieben, der eine Insertion aus cDNA enthält, die für das gespleißte virale Protein E6ˉ codiert:

HPV18-positive mRNA von HeLa-Zellen wurde in cDNA überführt und mit S1-Nuklease stumpfendig gemacht. Nach Ansetzen von EcoRI-Linkern und nach Schneiden mit EcoRI wurden die so erhaltenen Fragmente in den mit EcoRI geöffneten Phagen λNM1149 ligiert, verpackt und selektiert wie beschrieben (Murray, in: "Lambda II", R.W. Hendrix et al., eds., Seiten 359 - 432, Cold Spring Harbor Laboratory Press, New York, 1983). Die klonierte HPV18-DNA wurde mit EcoRI reisoliert und über Agarosegel-Elektrophorese gereinigt.

Die cDNA enthält am N-Terminus von E6 bzw. E6* eine Schnittstelle für NcoI. Durch Schneiden mit diesem Enzym und Auffüllen der überstehenden Enden erhält man ein stumpfendiges DNA-Fragment.

Der Expressionsvektor pEX 10-mer wird mit BamHI geschnitten und die überstehenden Enden aufgefüllt. Durch Ligieren des stumpfendig gemachten cDNA-Fragments in diese aufgefüllte Schnittstelle erhält man einen Vektor, der für ein Fusionsprotein codiert, in welchem auf die 100 Aminosäuren von MS2 zunächst die Aminosäuren Leu-Asp-Ser-Gly-Gly-Ser und dann über Methionin 56 Aminosäuren von E6* folgen.

In analoger Weise können weitere Expressionsvektoren konstruiert werden, die für die anderen viralen Proteine codieren.

Beispiel 2: Expression der Fusionsproteine

Kompetente Bakterien vom E. coli-Stamm 537 werden mit den Expressionsvektoren nach Beispiel 1 transformiert. Der Stamm enthält ein Plasmid-codiertes Repressorgen, dessen Temperatur-sensitives Genprodukt bei einer Temperatur von 28°C die vom λP$_L$-Promotor gesteuerte Expression hemmt. Die negative Regulation wird aufgehoben, wenn die Temperatur auf 42°C angehoben wird (Remaut et al., Gene 22, a.a.O.). Zusätzlich trägt das Plasmid das Resistenzgen für Ampicillin.

Einzelkolonien transformierter Bakterien werden zunächst bei 28°C bis zur stationären Phase angereichert und anschließend mit auf 42°C vorgewärmtem LB-Medium im Verhältnis 1 : 4 verdünnt und bei 42°C weitere 3 Stunden geschüttelt. Nach Pelletierung der Bakterien und Aufnahme eines Aliquots in Lyselösung können nach elektrophoretischer Analyse auf SDS-Polyacrylamidgelen die erwarteten Expressionsbanden identifiziert werden. Die Fusionsproteine machen bis zu 20 % des Gesamtzellproteins aus. Das Molekulargewicht der exprimierten Proteine stimmt mit dem von der Aminosäuresequenz abgeleiteten Wert gut überein.

Beispiel 3: Reinigung der Fusionsproteine

Die pelletierten E. coli-Zellen (5 l Kultur) werden in 40 ml Puffer (8 % Saccharose, 50 mM EDTA, 50 mM Tris, pH 8, 200 µg/ml Lysozym) resuspendiert und 15 Minuten bei Raumtemperatur belassen. Nach Zugabe von 0,1 % Tensid (®TRITON X-100, Polyoxyethylenprodukt) werden die Zellen beschallt und wie folgt weiterverarbeitet:

Nach 15 Minuten Inkubation bei 37°C zentrifugiert man 15 Minuten bei 18000 Umdrehungen pro Minute. Das Pellet wird in 25 ml PBS (8 mM Na$_2$HPO$_4$, 8 mM NaH$_2$PO$_4$, 140 mM NaCl) unter Zusatz von 0,1 % Tensid aufgenommen, beschallt, 15 Minuten bei 37°C inkubiert und wiederum 15 Minuten bei 18000 Umdrehungen pro Minute zentrifugiert. Das erhaltene Pellet wird in 25 ml 1 M Harnstoff aufgenommen, beschallt, 15 Minuten bei 37°C inkubiert und erneut 15 Minuten bei 18000 Umdrehungen pro Minute zentrifugiert. Das erhaltene Pellet wird in 7 M Harnstoff aufgenommen, beschallt und erneut 15 Minuten bei 18000 Umdrehungen pro Minute zentrifugiert. Der hierbei erhaltene Überstand erhält das bis zu 90 % angereicherte Fusionsprotein.

Beispiel 4: Immunisierungsschema

Kaninchen werden mit je 1 mg Fusionsprotein (dialysiert gegen PBS) und 1 ml Freunds komplettem Adjuvans immunisiert. Die Injektion gleicher Proteinmengen in Freunds inkomplettem Adjuvans unter die Haut wird in 3- bis 4-wöchigen Abständen mindestens 3mal wiederholt.

## Aufbereitung von Antiseren

Der geronnene Blutkuchen wird erst niedertourig (10 Minuten bei 5000 Umdrehungen pro Minute) zentrifugiert, das Serum (Überstand) abgenommen und das Pellet dann hochtourig (10 Minuten bei 15000 Umdrehungen pro Minute) nochmals zentrifugiert. Die Überstände werden vereinigt, mit Ammoniumsulfat zu 45 % gesättigt und für eine Stunde im Kühlraum stehengelassen. Das Präzipitat wird pelletiert (10 Minuten bei 12000 Umdrehungen pro Minute), in PBS gelöst und die Immunglobuline durch Ammoniumsulfat gefällt. Nach erneutem Pelletieren und Lösen des Pellets mit PBS werden die Antikörper über Nacht gegen PBS dialysiert, wobei der Puffer mehrmals gewechselt wird. Das Antiserum wird dann bei -20°C eingefroren.

## Reinigung der polyklonalen Antiseren (Affinitätschromatographie)

Da die Antiseren gegen Fusionsproteine aus E. coli gerichtet sind, enthalten sie auch Antikörper gegen den MS-2-Teil sowie gegen E. coli-Proteine. Um diese unerwünschten Antikörper zu eliminieren, werden MS-2-und E. coli-Proteine an Bromcyan-aktivierte ®SEPHAROSE gekoppelt wie nachfolgend beschrieben.

2 g Bromcyan-aktivierte SEPHAROSE werden mit 200 ml 1 mM eiskalter Salzsäure und anschließend mit 100 ml Kopplungspuffer (50 mM Borat, pH 8, 500 mM LiCl, 0,5 % SDS, ebenfalls eiskalt) über Glasfilter gewaschen, in 20 ml Proteinlösung (2 mg/ml, gegen Kopplungspuffer dialysiert) aufgenommen und 2 Stunden bei Raumtemperatur rotiert. Nicht gebundene Proteine werden mit Kopplungspuffer weggewaschen und freie Bindungskapazitäten durch Zugabe von 1 M Ethanolamin (pH 9) abgesättigt (2 Stunden bei Raumtemperatur). Die Protein-SEPHAROSE-Komplexe werden anschließend abwechselnd mit Kopplungs- und Acetatpuffer (0,1 M Acetat, pH 4, 500 mM NaCl) gewaschen, in eine Säule gefüllt und mit PBS äquilibriert.

Alle Antiseren werden je 4mal über eine solche Säule gegeben. Nach jedem Reinigungsschritt werden die gebundenen gegen MS-2 und E. coli Proteine gerichteten Antikörper durch Waschen mit 0,1 M Glycin (pH 2,5) eluiert, die Proteinsäule mit PBS neu äquilibriert und die Reinigungsprozedur wiederholt. Die Seren, die nun frei sind von Antikörpern gegen MS-2 und E. coli-Proteine, werden mit 0,1 % Natriumazid versetzt und bei 4°C aufbewahrt.

Mit Hilfe der Antiseren kann nicht nur in an sich bekannter Weise ein direkter Nachweis der Antigene erfolgen, sondern auch eine Anreicherung der Antigene mit Hilfe von Antikörpersäulen, die mit den erfindungsgemäß erhaltenen Antiseren hergestellt werden.

Mit den erhaltenen Fusionsproteinen können auch nach an sich bekannten Methoden monoklonale Antikörper hergestellt und in Diagnostika eingesetzt werden.

Die Bestimmung der Antikörper-Titer der erhaltenen spezifischen Antiseren erfolgte im "Western Blot"-Test gegen die jeweiligen Fusionsproteine. Hierzu wurden die Antigene in einer Verdünnungsreihe eingestellt, die Verdünnungen auf ein SDS-Polyacrylamidgel gegeben, die Proteine nach der Elektrophorese auf Nitrocellulose transferiert und die Filter mit einer Verdünnung des entsprechenden Antiserums über Nacht inkubiert. Nach Wegwaschen ungebundener Antikörper, Inkubation jedes Filters in 10 cpm $^{125}$J-Protein A für mindestens 2 Stunden wurde die Waschprozedur wiederholt, die Filter getrocknet und damit Röntgenfilme exponiert. Folgende Titer wurden gefunden:

| Fusionsprotein mit | Exposition, h | ng/Bande |
|---|---|---|
| HPV16 E6 | | |
| HPV18 E6 | | |
| HPV16 L1, N-terminaler Teil | 3 | 10 |
| HPV16 L1, C-terminaler Teil | 24 | 1 |
| HPV16 E7 | | |
| HPV18 E7 | 3 | 1 |
| HPV16 E4 | | |
| HPV18 E1 | | |

6

DNA-Sequenz I

1 ATACTATACTTTCGTTAATACTTTTAACAATTGTAGTATATAAAAAAGGGAGTGACCGAAAACGGTCGGGACCGAAAACGGTGTATATAAAAGATGTGAG

E6 ———→

101 AAACACACCACAATACTATCGCCGCGCTTTGAGGATCCAACACGGCGACCCTACAAGCTACCTGATCTCTGCACGGAACTGAACACTTCACTGCAAGACAT
   MetAlaArgPheGluAspProThrArgArgProTyrLysLeuProAspLeuCysThrGluLeuAsnThrSerLeuGlnAspIle     R1

201 AGAAATAACCTGTGTATATTGCAAGACAGTATTGGAACTTACAGAGGTATTTGAATTTGCATTTAAAGATTTATTTGTGGTGTATAGAGACAGTATACCG
   GluIleThrCysValTyrCysLysThrValLeuGluLeuThrGluValPheGluPheAlaPheLysAspLeuPheValValTyrArgAspSerIlePro     R1

301 CATCCTGCATGCCATAAATGTATAGATTTTTATTCTAGAATTAGAGAATTAAGACATTATTCAGACTCTGTGTATGGAGACACATTGGAAAAACTAACTA
   HisAlaAlaCysHisLysCysIleAspPheTyrSerArgIleArgGluLeuArgHisTyrSerAspSerValTyrGlyAspThrLeuGluLysLeuThrAsn     R1

401 ACACTGGGTTATACAATTTATTAATAAGGTGCCTGCGGTGCCAGAAACCGTTGAATCCAGCAGAAAAACTTAGACACCTTAATGAAAAACGACGATTTCA
   ThrGlyLeuTyrAsnLeuLeuIleArgCysLeuArgCysGlnLysProLeuAsnProAlaGluLysLeuArgHisLeuAsnGluLysArgArgPheHis     F1

501 CAACATAGCTGGGCACTATAGAGGCCAGTGCCATTCGTGCTGCAACCGAGCACGACAGGAACGACTCCAACGACGCAGAGAAACACAAGTATAATATTAA
   AsnIleAlaGlyHisTyrArgGlyGlnCysHisSerCysCysAsnArgAlaArgGlnGluArgLeuGlnArgArgArgGluThrGlnVal✷✷✷     R1

E7 ———→

601 GTATGCATGGACCTAAGGCAACATTGCAAGACATTGTATTGCATTTAGAGCCCCCAAAATGAAATTCCGGTTGACCTTCTATGTCACGAGCAATTAAGCGA
   MetHisGlyProLysAlaThrLeuGlnAspIleValLeuHisLeuGluProGlnAsnGluIleProValAspLeuLeuCysHisGluGlnLeuSerAsp     P3

701 CTCAGAGGAAGAAAACGATGAAATAGATGGAGTTAATCATCAACATTTACCAGCCCGACGAGCCGAACCACAACGTCACACAATGTTGTGTATGTGTTGT
   SerGluGluGluAsnAspGluIleAspGlyValAsnHisGlnHisLeuProAlaArgArgAlaGluProGlnArgHisThrMetLeuCysMetCysCys     P3

801 AAGTGTGAAGCCAGAATTGAGCTAGTAGTAGAAAAGCTCAGCAGACGACCTTCGAGCATTCCAGCAGCTGTTTCTGAACACCCTGTCCTTTGTGTGTCCGT
   LysCysGluAlaArgIleGluLeuValValGluSerSerAlaAspAspLeuArgAlaPheGlnGlnLeuPheLeuAsnThrLeuSerPheValCysProTrp     P3

E1 ———→

901 GGTGTGCATCCCAGCAGTAAGCAACAATGGCTGATCCAGAAGGTACAGACGGGGAGGGCACGGGTTGTAACGGCTGGTTTTATGTACAAGCTATTGTAGA
   CysAlaSerGlnGln✷✷✷     MetAlaAspProGluGlyThrAspGlyGluGlyThrGlyCysAsnGlyTrpPheTyrValGlnAlaIleValAsp     R3

DNA-Sequenz I ff

1001 CAAAAAAACAGGAGATGTAATATCAGATGACGAGGACGAAAATGCAACAGACACAGAGGGTCGGATATGGTAGATTTTATTGATACACAAGGAACATTTTGT
LysLysThrGlyAspValIleSerAspAspGluAspGluAsnAlaThrAspThrGlySerAspMetValAspPheIleAspThrGlnGlyThrPheCys     R3

1101 GAACAGGCAGAGCTAGAGACAGCCACAGGCATTGTTCCATGCGCAGGAGGTCCACAATGATGCCACAAGTGTTGCATGTTTTAAAACGAAAGTTTGCAGGAG
GluGlnAlaGluLeuGluThrAlaGlnAlaLeuPheHisAlaGlnGluValHisAsnAspAlaGlnValLeuHisValLeuLysArgLysPheAlaGlyGly   R3

1201 GCAGCACAGAAAACAGTCCATTAGGGGAGCGGCTGGAGGTGGATACAGAGTTAAGTCCACGGTTACAAGAAATATCTTTAAAATAGTGGGCAGAAAAAGGC
SerThrGluAsnSerProLeuGlyGluArgLeuGluValAspThrGluLeuSerProArgLeuGlnGluIleSerLeuAsnSerGlyGlnLysLysAla    R3

1301 AAAAAGGCGGCTGTTTACAATATCAGATAGTGGCTATGGCTGTTCTGAAGTGGAAGCAACACAGATTCAGGTAACTACAAATGGCGAACATGGCGGCAAT
LysArgArgLeuPheThrIleSerAspSerGlyTyrGlyCysSerGluValGluAlaThrGlnIleGlnValThrThrAsnGlyGluHisGlyGlyAsn    R3

1401 GTATGTAGTGGCGGCAGTACGGAGGCTATAGACAACGGGGGCACAGAGGGCAACAACAGCACTGTAGACGGTACAAGTGACAATAGCAATATAGAAAATG
ValCysSerGlyGlySerThrGluAlaIleAspAsnGlyGlyThrGluGlyAsnAsnSerSerValAspGlyThrSerAspAsnSerAsnIleGluAsnVal   R3

1501 TAAATCCACAATGTACCATAGCACAATTAAAAGACTTGTTAAAAGTAAACAATAAACAAGGAGCTATGTTAGCAGTATTTAAAGACACATATGGGCTATC
AsnProGlnCysThrIleAlaGlnLeuLysAspLeuLeuLysValAsnAsnLysGlnGlyAlaMetLeuAlaValPheLysAspThrTyrGlyLeuSer    R3

1601 ATTTACAGATTTAGTTAGAAATTTTAAAAGTGATAAAACCACGTGTACAGATTGGGTTACAGCTATATTTGGAGTAAACCCAACAATAGCAGAAGGATTT
PheThrAspLeuValArgAsnPheLysSerAspLysThrThrCysThrAspTrpValThrAlaIlePheGlyValAsnProThrIleAlaGluGlyPhe    R3

1701 AAAACACTAATACAGCCATTTATATTATATGCCCATATTCAATGTCTAGA
LysThrLeuIleGlnProPheIleLeuTyrAlaHisIleGlnCysLeu                                                        R3

## DNA-Sequenz II

0 256 321

E6
E6*

```
  1 TACTTTTAACAATTGTAC TATATAA AAAAGGGAGTG ACCC AAAA GGT CGGG ACCG AAAA CGGT G TATATAA AAGATGTGAGAAACACACCACAATACCA
                                                                                           ▼▼ ▼▼ ▼▼
                                                                                        Met  R1
                                                                                        Met  R1

        Bam HI
101 TGGCGCGCTTTGAGGATCCAACACGGCGACCCTACAAGCTACCTGATCTGTGCACGGAACTGAGCACTTCACTGCAAGACATAGAAATAACCTGTGTATA
      AlaArgPheGluAspProThrArgArgProTyrLysLeuProAspLeu Cys ThrGluLeuSerThrSerLeuGlnAspIleGluIleThr Cys ValTyr  R1
      AlaArgPhe  LysAspProThrArgArgProTyrLysLeuProAspLeu Lys ThrGluLeuSerThrSerLeuGlnAspIleGluIleThr  ys ValTyr  R1

                                          ↓
201 TTGCAAGACAGTATTGGAACTTACAGAGGTATTTGAATTTGCATTTAAAGATTTATTTGTGGGTGTATAGAGACAGTATACCGCATGCTGCATGCCATAAA
      Cys LysThrValLeuGluLeuThrGluValPheGluPheAlaPheLysAspLeuPheValValTyrArgAspSerIleProHisAlaAla Cys HisLys  R1
       ys LysThrValLeuGluLeuThrGlu------                                                                      R1

301 TGTATAGATTTTTATTCTAGAATTAGAGAATTAAGACATTATTCAGACTCTGTGTATGGAGACACATTGGAAAAACTAACTAACACTGGGTTATACAATT
      Cys IleAspPheTyrSerArgIleArgGluLeuArgHisTyrSerAspSerValTyrGlyAspThrLeuGlu LysLeuThrAsnThrGlyLeuTyrAsnLeu  R1

                        ↓
401 TATTAATAAGGTGCCTGCGGTGCCAGAAACCGTTGAATCCAGCAGAAAAACTTAGACACCTTAATGAAAAACGACGATTTCACAACATAGCTGGGCACTA
      LeuIleArg Cys LeuArg Cys GlnLysProLeuAsnProAlaGluLysLeuArgHisLeuAsnGluLysArgArgPheHisAsnIleAlaGlyHisTyr  R1
      ----------ValProAlaValProGluThrValGluSerSerArgLysThr                                                  R3

                                                                                E7
501 TAGAGGCCAGTGCCATTCGTGCTGCAACCGAGCACGACAGGAAAGACTCCAACGACGCAGAGAAACACAAGTA TAATATT AAGT ATGCATGGACCTAAGG
      ArgGlyGln LysHisSer Cys Cys AsnArgAlaArgGlnGluArgLeuGlnArgArgArgGluThrGlnVal                               R1
                                                                                         MetHisGlyProLysAla  R3

601 CAACATTGCAAGACATTGTATTGCATTTAGAGCCCCAAAATGAAATTCCGGTTGACCTTCTATGTCACGAGCAATTAAGCGACTCAGAGGAAGAAAACGA
      ThrLeuGlnAspIleValLeuHisLeuGluProGlnAsnGluIleProValAspLeuLeu Cys HisGluGlnLeuSerAspSerGluGluGluAsnAsp   R3

701 TGAAATAGATGGAGTTAATCATCAACATTTACCAGCCCGACGAGCTGAACCACAACGTCACACAATGTTGTGTATGTGTTGTAAGTGTGAAGCCAGAATT
      GluIleAspGlyValAsnHisGlnHisLeuProAlaArgArgAlaGluProGlnArgHisThrMetLeu Cys Met Cys Cys Lys Cys GluAlaArgIle  R3

801 AAGCTAGTAGTAGAAAAGCTCAGCAGACGACCTTCGAGCATTCCAGCAGCTGTTTCTGAACACCCTGTCCTTTGTGTGTCCGTGGTGTGCATCCCAGCAGT
      LysLeuValValGluSerSerAlaAspAspLeuArgAlaPheGlnGlnLeuPheLeuAsnThrLeuSerPheVal Cys ProTrp Cys AlaSerGlnGln   R3

       E1                    ↓
901 AAGCAACAATGGCTGATCCAGAAGGTACAGACGGGGAGGGCACGGGTTGTAACGGCTGGTTTTATGTACAAGCTATTGTAGACAAAAAAACAGGAGATGT
```

**Ansprüche**

1. HPV18 DNA-Sequenz I.

2. Gentechnisch erhältliche Expressionsprodukte von HPV18.

3. HPV18 E1-, E6-, E6'-, E7-und L1-Protein.

4. Verwendung der Proteine nach Anspruch 2 und 3 zur Herstellung von Antikörpern.

5. Mono-und polyklonale Antikörper gegen Expressionsprodukte von HPV18.

6. Verwendung der Antikörper nach Anspruch 5 zur Herstellung von Diagnostika.

7. Diagnostika, gekennzeichnet durch einen Gehalt an einem Antikörper nach Anspruch 5.

8. Verfahren zur Diagnose von HPV18-Infektionen, dadurch gekennzeichnet, daß man Biopsie-oder Abstrichmaterial mit einem Diagnostikum nach Anspruch 7 in Kontakt bringt.

9. Verwendung der für die Proteine nach Anspruch 2 und 3 codierenden DNA zur gentechnischen Herstellung dieser Proteine.

10. Verwendung der für die Proteine nach Anspruch 2 und 3 codierenden DNA oder Teilen davon zur Diagnose von HPV18-Infektionen.

11. Diagnostikum, enthaltend DNA, die für die Proteine nach Anspruch 2 und 3 codiert, oder Teile dieser DNA.

12. Verfahren zur Diagnose von HPV18-Infektionen, dadurch gekennzeichnet, daß man die zu untersuchende RNA oder DNA mit DNA hybridisiert, die für die Proteine nach Anspruch 1 oder 2 oder für Teile dieser Proteine codiert.

13. Expressionsvektoren pEX 8-mer, pEX 10-mer und pEX 12-mer.

14. Hybridvektoren, die in einem der Vektoren nach Anspruch 13 zu exprimierende DNA im Polylinkerteil enthalten.

15. Verwendung der Produkte nach Anspruch 2 zur Vakzinierung und Therapie.

16. Verwendung der Produkte nach Anspruch 1 zur Phophylaxe und Therapie.

## EUROPÄISCHER TEILRECHERCHENBERICHT,

**Europäisches Patentamt**

der nach Regel 45 des Europäischen Patentübereinkommens für das weitere Verfahren als
europäischer Recherchenbericht gilt

Nummer der Anmeldung

EP 87 11 0347

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | UCLA SYMP. MOL. CELL. BIOL., New Ser. Band 32, 1985, "Papillomaviruses: Molecular and clinical aspects"; Seiten 391-396; A.R. Liss Inc.<br>K. SEEDORF et al.: "Human papillomavirus type 16 DNA: Expression of open reading frames in E. coli."<br><br>* Insgesamt *<br><br>--- | 1-14 | C 12 N 15/00<br>C 12 Q 1/70<br>C 12 P 21/02<br>C 12 P 21/00<br>G 01 N 33/569<br>G 01 N 33/571<br>A 61 K 39/12 |
| Y,D | CHEMICAL ABSTRACTS, Band 101, 1984, Seite 166, Ref.Nr. 18345e; Columbus, Ohio, US<br>M. BOSHART et al.: "A new type of papillomavirus DNA, its presence in genital cancer biopsies and in cell lines derived from cervical cancer."<br>& EMBO J. 1984, 3(5), 1151-7.<br><br>* Zusammenfassung *<br><br>--- | 1-14 | RECHERCHIERTE SACHGEBIETE (Int. Cl.4)<br><br>C 12 N<br>C 12 Q |
| Y | NATURE, Band 314, 7. März 1985, Seiten 111-116 | | |

./.

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-14

Unvollständig recherchierte Patentansprüche:

Nicht recherchierte Patentansprüche: 15,16

Grund für die Beschränkung der Recherche:

Verfahren zur chirurgischen oder therapeutischen Behandlung des menschlichen oder tierischen Körpers (Siehe Art. 52(4) des Europäischen Patentübereinkommens).

| Recherchenort | Abschlußdatum der Recherche | Prüfer |
|---|---|---|
| Den Haag | 18-09-1987 | SKELLY |

| | EINSCHLÄGIGE DOKUMENTE | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| | E. SCHWARZ et al.: "Structure and transcription of human papilloma-virus sequences in cervical carci-noma cells." * Seite 112, Spalte 1, Zeilen 9,10, 31-38, Figur 1, Spalte 2, Zeilen 20-28; Seite 113, Spalte 1, Zeilen 1-11 * --- | 1-14 | |
| Y | EP-A-0 133 123 (MOLECULAR GENETICS INC.) * Ansprüche * --- | 1-14 | |
| P,X | WO-A-86 05 816 (GEORGETOWN UNIV.) * Seite 12, Zeile 16 - Seite 14, Zeile 1, Zeile 24 - Seite 15, Zeile 25; Ansprüche * --- | 1-14 | RECHERCHIERTE SACHGEBIETE (Int. Cl. 4) |
| P,X | CHEMICAL ABSTRACTS, Band 106, Nr. 17, 27. April 1987, Seite 152, Ref.Nr. 132597s; Columbus, Ohio, US K. SEEDORF et al.: "Identification of early proteins of the human papilloma viruses type 16 (HPV 16) and type 18 (HPV 18) in cervical carcinoma cells." & EMBO J. 1987, 6(1), 139-44. * Zusammenfassung * --- | 1-14 | |
| P,X | CHEMICAL ABSTRACTS, Band 106, Nr. 1, 5. Januar 1987, Seite 112, Ref. Nr. 1115k; Columbus, Ohio, US G. MATLASHEWSKI et al.: "The ex-pression of human papillomavirus type 18 E6 protein bacteria and the production of anti-E6 antibo-dies." & J. GEN. VIROL. 1986, 67(9), 1909-16. * Zusammenfassung * --- | 1-14 | |
| P,X | CHEMICAL ABSTRACTS, Band 106, 1987, Seite 140, Ref.nr. 61998k; Columbus, Ohio, US ./.. | | |

EPA Form 1505.3  06.78

| **EINSCHLÄGIGE DOKUMENTE** | | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| | E. SCHWARZ et al.: "Expression of human papillomavirus type-18 DNA in cervical carcinoma cell lines." & CANCER CELLS 1986, 4(DNA Tumor Viruses: Control Gene Expression Replication), 581-7.<br><br>* Zusammenfassung *<br><br>--- | <br><br><br><br><br><br><br>1 | |
| P,X | EP-A-0 192 001 (INST. PASTEUR)<br><br>* Seite 31, Zeilen 9-32; Ansprüche *<br><br>--- | <br><br>1-14 | |
| E | WO-A-87 01 375 (INST. PASTEUR)<br><br>* Ansprüche *<br><br>--- | <br><br>1-14 | RECHERCHIERTE SACHGEBIETE (Int. Cl. 4) |
| A | EP-A-0 092 456 (INST. PASTEUR)<br><br>-------------- | | |

EPA Form 1505.3   06.78